(19) Europäisches Patentamt
European Patent Office
Office européen des brevets

(11) Publication number: **0 228 795 B1**

# (12) EUROPEAN PATENT SPECIFICATION

(45) Date of publication of patent specification: **06.02.91**

(51) Int. Cl.$^5$: **C 07 D 401/12**, A 61 K 31/445

(21) Application number: **86308899.3**

(22) Date of filing: **14.11.86**

## (54) Piperidine derivatives having a psychotropic activity.

(30) Priority: **15.11.85 GB 8528235**

(43) Date of publication of application:
**15.07.87 Bulletin 87/29**

(45) Publication of the grant of the patent:
**06.02.91 Bulletin 91/06**

(84) Designated Contracting States:
**AT BE CH DE ES FR GR IT LI LU NL SE**

(56) References cited:
**EP-A-0 007 525**
**EP-A-0 035 374**
**FI-A- 77 231**
**GB-A-2 034 305**
**GB-A-2 106 108**
**US-A-4 199 590**

**The file contains technical information submitted after the application was filed and not included in this specification**

(73) Proprietor: **JOHN WYETH & BROTHER LIMITED**
**Huntercombe Lane South Taplow**
**Maidenhead Berkshire, SL6 0PH (GB)**

(72) Inventor: **Archibald, John Leheup, Dr.**
**Koinonia Black Pond Lane**
**Farnham Royal Buckinghamshire (GB)**
Inventor: **Ward, Terence James, Dr.**
**43 Loosen Drive**
**Maidenhead Berkshire (GB)**

(74) Representative: **Wileman, David Francis Dr. et al**
**c/o Wyeth Laboratories Huntercombe Lane South**
**Taplow Maidenhead Berkshire SL6 OPH (GB)**

**EP 0 228 795 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to piperidine derivatives, to processes for preparing them and to pharmaceutical compositions containing them.

In our GB—2073176B (see also EP-35374A), there are described and claimed a class of piperidine derivatives which exhibit psychotropic activity in standard pharmacological test procedures and are potentially useful as antidepressants. In general the compounds are specific inhibitors of 5-hydroxytryptamine re-uptake *in vitro* and *in vivo*, and therefore are also useful in any other therapeutic applications where such pharmacological specificity is beneficial. The piperidine derivatives of GB—2073176B have the formula (II)

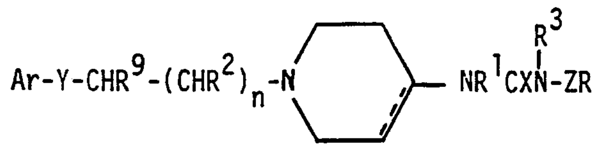

(II)

and acid addition and quaternary ammonium salts thereof, wherein the dotted line represents an optional bond, Ar represents a ring system of formula.

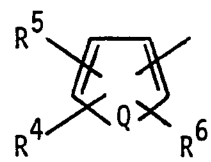

in which Q is O, S, —$CR^7$=$CR^8$—, —N=$CR^8$— and —N=N—; $R^4$, $R^5$ and $R^6$, and $R^7$ and $R^8$ when present, each represent hydrogen or a substituent selected from halogen, lower alkyl, lower alkenyl, lower alkoxy, $NO_2$, $NH_2$, haloloweralkyl, hydroxyloweralkyl, aminoloweralkyl, substituted amino, loweralkoxycarbonyl, cyano, $CONH_2$ and hydroxy; and additionally either $R^4$ and $R^5$ when adjacent or $R^6$ and $R^8$ when adjacent, together with the carbon atoms to which they are attached also represent a fused five or six membered carbocyclic or heterocyclic ring optionally carrying one or more substituents as defined above; R is an optionally substituted aryl or heteroaryl radical or a cycloalkyl radical containing 5 to 7 carbon atoms; $R^1$, $R^2$, $R^3$ and $R^9$ are each hydrogen or a lower alkyl group; n is 0 or 1; X is =O or =S; Y is —O— or a direct bond and Z is —CO— or —$CH_2$— with the provisos that (i) when Ar is unsubstituted phenyl and $R^9$ is hydrogen then Y is —O— and (ii) when Z is $CH_2$ and Ar represents phenyl or pyridyl group either of which may be substituted then $R^1$ is hydrogen.

The term 'lower' as used in connection with alkyl or alkoxy groups means that such groups contain 1 to 6 carbon atoms. 'Substituted amino' includes groups such as alkyl- or a dialkyl-amino, acylamino e.g. lower alkylcarbonylamino, ureido or sulphonylamino, e.g. lower alkylsulphonamido or di-lower-alkylsulphonylamino.

Pharmaceutical compositions comprising compounds of formula (II) are claimed in our GB—2108489B.

The compounds of formula II were tested for psychotropic activity by their ability to inhibit p-chloroamphetamine (pCA) induced hyperactivity and/or by their ability to inhibit 5-hydroxytryptamine (5-HT) re-uptake in brain slices.

Example 42 of EP-35374 describes the compound 1-(pyrid-4-oyl)-3-[1-naphth-2-yl(methyl)piperid-4-yl]urea. Example 42 of Finnish Patent Application No. 810587 as open to public inspection describes the compound 1-(pyrid-3-oyl)-3-[1-(naphth-2-ylmethyl)piperid-4-yl]urea (FI—77231).

We have now surprisingly found a small class of compounds, not specifically disclosed in the abovementioned specifications, which are extremely potent inhibitors of pCA induced syndrome.

Accordingly this invention provides compounds of formula:

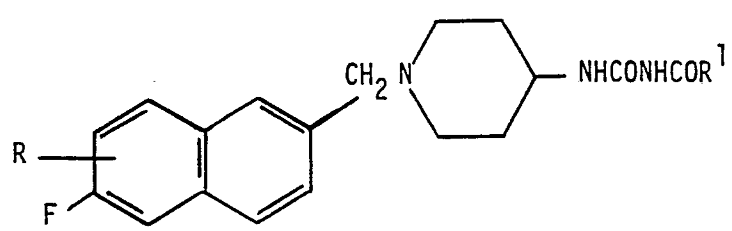

or salts thereof, wherein $R^1$ is 3- or 4-pyridyl, R is hydrogen or fluorine, and is bonded to any of the vacant naphthalene ring positions, e.g. 5- or 7-. In formula I, R is preferably hydrogen in which case the compound is N - [[[1 - [(6 - fluoro - 2 - naphthyl)methyl] - 4 - piperidinyl]amino]carbonyl] - 3 - pyridinecarboxamide (A) or N - [[[1 - [6 - fluoro - 2 - naphthyl)methyl] - 4 - piperidinyl]amino]carbonyl] - 4 - pyridine carboxamide (B).

Representative compounds of the invention were tested for ability to inhibit pCA induced syndrome in rats by the standard procedure described below:

Inhibition of p-chloroamphetamine (pCA)-induced stereotypy

Vehicle or drug (5 dose levels) were administered p.o. to six groups of 6—8 male Sprague-Dawley rats (300—400 g) followed, 90 minutes later, by pCA (10 mg/kg i.p.). The animals were then placed in individual observation chambers and, 30 minutes after pCA administration, the intensity of the pCA-induced 5-HT syndrome was assessed according to the following scoring system:

| | |
|---|---|
| hind-limb abduction<br>head-weaving | 0, 1, 2 or 3 according to severity |
| fore-paw treading<br>tremor | 0 (absent) or 1 (present) |

Therefore, the maximum score for each animal was 10.

The inhibition of pCA induced stereotypy is calculated for each dose level as follows:

$$\frac{C-T}{C} 100\%$$

where

C = control group total score at 30 minutes post pCA.

T = group total score of treated group at 30 minutes post pCA.

For each dose a % effect is calculated.

The results obtained from the tests using 5 different dose levels of the drug allow the $ED_{50}$ value (i.e. the dose required to produce 50% inhibition of pCA induced stereotypy) to be calculated.

In the aforementioned test the compounds A and B antagonised pCA-induced stereotypy in a dose-dependent manner.

The test was carried out using the compound A in free base form and also as the mono hydrochloride salt. Compound B was used in the form of the succinic acid salt.

The $ED_{50}$ values obtained were: A 2.5 mg/kg (free base) and 2.3 mg/kg (monohydrochloride, corrected for amount of active ingredient) and B 3.8 mg/kg (corrected for amount of active ingredient). At 50 mg/kg compound A showed a 99% inhibition of syndrome. These values are markedly lower than any values found for related compounds disclosed in the specification of UK Patent Publication No. 2073176B.

In the same test the most preferred compound from UK Patent Publication No. 2073176B namely, 1-benzoyl-3-[1-(naphth-2-ylmethyl)piperid-4-yl]urea (which has the generic name panuramine) had an $ED_{50}$ of 16.2 mg/kg (monohydrochloride corrected for amount of active ingredient). At 50 mg/kg panuramine showed a ca. 78% inhibition of syndrome.

The compound (A) was also tested for its ability to potentiate 5-hydroxy-L-tryptophan induced behavioural syndrome in rats. The test procedure is described below (updated from that described in UK 207317B).

Potentiation of 5-hydroxytryptophan (5-HTP)-induced behaviour

Groups of 10 male Sprague-Dawley rats (310—360 g) were dosed p.o. with vehicle or drugs. Ninety minutes later 5-HTP (50 mg/kg s.c.) was administered and the animals placed in individual observation chambers (peripheral decarboxylation was prevented by 25 mg/kg i.p. carbidopa administered 60 minutes before 5-HTP). Head shakes were counted over the period 30—45 minutes after 5-HTP and the intensity of the 5-HT syndrome was scored immediately afterwards using the system described for the pCa procedure above. Percentage potentiation of syndrome was calculated as follows:

| | |
|---|---|
| hind-limb abduction head-weaving | 0 1, 2 or 3 according to severity |
| tremor fore-paw treading | 0 (absent) or 1 (present) |

Percentage potentiation was calculated from the following:

$$\frac{\text{test score} - \text{control score}}{\text{maximum possible score} - \text{control score}} \times 100$$

In this test the compound (A) as the citrate salt had an $ED_{50}$ value of 5.0 mg/kg (corrected for amount of base).

This value is also markedly lower than the value found for panuramine HCl salt which in the same test had an $ED_{50}$ value of 27.4 mg/kg (corrected for amount of base).

In vitro tests have shown that compounds of formula I also have a marked degree of selectivity in inhibiting uptake of 5-HT into rat brain synaptosomes relative to uptake of $^3H$ noradrenaline. The test procedure involved obtaining synaptosomal preparations from male Sprague Dawley rats according to the method of Grey and Whittaker* as modified by Wood and Wyllie**. Aliquots of the synaptosomal preparation were then incubated with tritrated noradrenaline (NA) or 5-HT at a temperature of 37°C for 4 minutes. The active synaptosomal accumulation of labelled substrate was measured by filtration and scintillation counting. The effect at a range of concentrations of test compound enabled $IC_{50}$ values and selectivity ratios to be calculated. The values found for compound A and panuramine are shown below:

| | $IC_{50}$ (μM) | | |
|---|---|---|---|
| Compound | 5-HT uptake | NA | Selectivity Ratio |
| A | 0.044 | 20.0 | 455 |
| panuramine | 0.063 | 8.5 | 135 |

*Grey and Whittaker, — J. Anat. *96* 79 (1962)
**Wood and Wyllie, J. Neurochemistry, *37*, 795 (1981)

The compounds of the present invention can be prepared by any of the appropriate general procedures described in our GB—2073176B.

In particular the compounds of the present invention can be prepared by reacting a compound of formula III

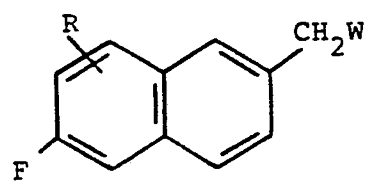

(III)

wherein R is as defined above and W represents a leaving group such as halogen (e.g. chlorine, bromine or iodine), an organic sulphonyloxy radical (e.g. tosyloxy, mesyloxy) or a radical of formula $—OSO_2OR^2$ where $R^2$ is

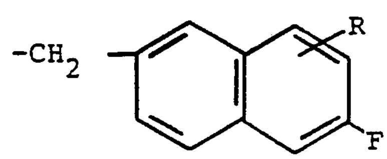

with a compound of formula IIIa

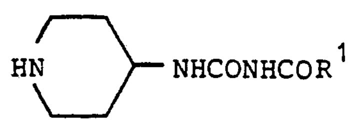

(IIIa)

wherein $R^1$ is as defined above.

This reaction is preferably carried out in the presence of base, e.g. an alkali metal carbonate such as $K_2CO_3$ or an amine such as triethylamine or diisopropylethylamine. Otherwise the reaction may be carried out by heating in the presence of an inert solvent, e.g. toluene.

A second method for preparing the compounds of this invention comprises reacting a compound of formula IV

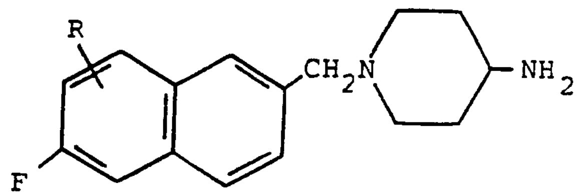

(IV)

wherein R is as defined above with a 3- or 4-pyridoylisocyanate. This reaction is conveniently carried out at room temperature and in an inert solvent. The starting material (IV) may be prepared by processes described in GB—1,345,872.

A further process for preparing the compounds of this invention comprises reacting the starting material IV with 3- or 4-pyridoylurea. Conveniently this reaction is carried out in the presence of a suitable inert solvent, for example, toluene, pyridine, xylene, chlorobenzene dimethylformamide or dioxan; pyridine being preferred. Preferably the reaction is carried out by heating at reflux until complete.

A still further process for preparing the compounds of this invention comprises acylating a compound of formula V

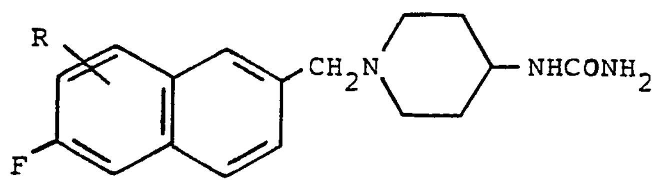

(V)

wherein R is as defined above, with an acylating agent containing the group —COR[1] wherein R[1] is as defined above.

Examples are reactive derivatives of pyridine-3 or 4-carboxylic acid such as the acid anhydride, mixed anhydride, acid halide or activated ester such as used in peptide chemistry. Other methods of acylation are well known in the art such as those employing coupling reagents such as carbodiimides, e.g. dicyclohexylcarbodiimide.

The compounds of this invention may also be prepared by reducing a corresponding compound of formula VI or VII

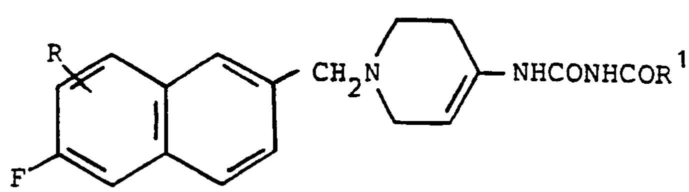

(VI)

or

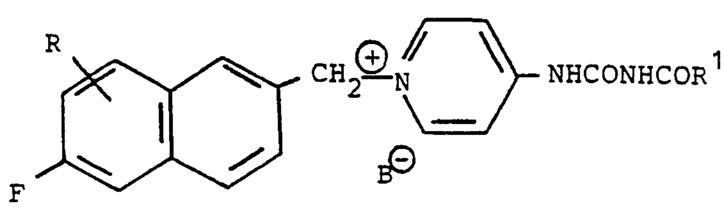

(VII)

wherein B represents an anion, e.g. a halide ion. For example catalytic hydrogenation e.g. in the presence of Raney nickel or platinum catalyst gives the compound of the invention. The reduction may also be effected by a process described and claimed in our GB—1542137. Such a reduction process employs an alkali metal borohydride in a secondary alkanol having 3—5 carbon atoms, e.g. isopropanol. Alternatively reduction of the compound of formula VII using an alkali metal borohydride in methanol gives the dehydropiperidine compound of formula VI.

Yet a further process for preparing the compounds of this invention comprises reacting a compound of formula III wherein W is hydroxy with a compound of formula IIIa in the presence of catalyst, e.g. a nickel catalyst such as Raney nickel.

In any of the aforementioned processes the compounds of the invention may be isolated in free base form or as salts e.g. an acid addition salt. Quaternisation of the tertiary nitrogen of the piperidine ring may be included as an optional after step, e.g. using alkyl or aryl lower alkyl halides, e.g. methyl iodide, benzyl chloride.

Acid addition salts include salts with pharmaceutically acceptable acids such as the hydrochloric, sulphuric, nitric, hydrobromic, hydroiodic, acetic, citric, tartaric, phosphoric, fumaric, malonic, formic and maleic acid addition salts.

This invention further provides a pharmaceutical composition comprising a compound of formula I or a pharmaceutically acceptable salt thereof in association with a pharmaceutically acceptable carrier. Any suitable carrier known in the art can be used to prepare the pharmaceutical composition. In such a composition, the carrier is generally a solid or liquid or a mixture of a solid and a liquid.

Solid form compositions include powders, granules, tablets, capsules (e.g. hard and soft gelatin capsules), suppositories and pessaries. A solid carrier can be, for example, one or more substances which may also act as flavouring agents, lubricants, solubilisers, suspending agents, fillers, glidants, compression aids, binders or tablet-disintegrating agents; it can also be an encapsulating material. In powders the carrier is a finely divided solid which is in admixture with the finely divided active ingredient. In tablets the active ingredient is mixed with a carrier having the necessary compression properties in suitable proportions and compacted in the shape and size desired. The powders and tablets preferably contain up to 99%, e.g. fro 0.03 to 99%, preferably 1 to 80% of the active ingredient. Suitable solid carriers include, for example, calcium phosphate, magnesium stearate, talc, sugars, lactose, dextrin, starch, gelatin, cellulose, methyl cellulose, sodium carboxylmethyl cellulose, polyvinylpyrrolidine, low melting waxes and ion exchange resins.

The term "composition" is intended to include the formulation of an active ingredient with encapsulating material as carrier to give a capsule in which the active ingredient (with or without carriers) is surrounded by the carrier, which is thus in association with it. Similarly cachets are included.

Liquid form compositions include, for example, solutions, suspensions, emulsions, syrups, elixirs and pressurised compositons. The active ingredient, for example, can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilisers, emulsifiers, buffers, preservatives, sweeteneers, flavouring agents, suspending agents, thickening agents, colours, viscosity regulators, stabilisers or osmo-regulators. Suitable examples of liquid carriers for oral and parenteral administration include water (particularly containing additives as above e.g. cellulose derivatives, preferably sodium carboxymethyl cellulose solution, alcohols (including monohydric alcohols and polyhydric alcohols e.g. glycerol and glycols) and their derivatives, and oils (e.g. fractionated coconut oil and arachis oil). For parenteral administration the carier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilised by, for example, intramuscular, intraperitoneal or subcutaneous injection. Sterile solutions can also be administered intravenously. When the compound is orally active it can be administered orally either in liquid or solid composition form.

Preferably the pharmaceutical composition is in unit dosage form, e.g. as tablets or capsules. In such form, the comosition is sub-divided in unit doses containing appropriate quantities of the active ingredient; the unit dosage forms can be packaged compositions, for example packeted powders, vials, ampoules, prefilled syringes or sachets containing liquids. The unit dosage form can be, for example, a capsule or tablet itself, or it can be the appropriate number of any such compositions in package form. The quantity of the active ingredient in a dose of composition may be varied or adjusted from 0.5 mg or less to 750 mg or more, according to the particular need and the activity of the active invention. The invention also includes the compounds in the absence of the carrier where the compounds are in unit dosage form.

The following Example illustrates the invention:

## Example 1

*N*[[[1-[(6-Fluoro-2-naphthyl)methyl]-4-piperidinyl]amino]carbonyl]-3-pyridinecarboxamide

A solution of 2-bromomethyl-6-fluoronaphthalene (99 g) in dimethylformamide (400 ml) was added over 1 hour to a stirred suspension *N*[[[4-piperidinyl]amino]carbonyl-3-pyridinecarboxamide (102.5 g) in dimethylformamide (1000 ml) and diisopropylethylamine (72 ml). After stirring for a further hour the reaction was diluted with water and the precipitated product collected, washed with water, then ether and dried to give 132 g (78.5%). The base was dissolved in chloroform (700 ml) and added to a solution of maleic acid (40 g) in ethanol (400 ml) to precipitate the maleate salt of the title compound 142 g (65.8%) m.p. 206—207°C.

Analysis

$C_{23}H_{23}N_4FO_2 \cdot C_4H_4O_4$ requires: C, 62.1; H, 5.2; N, 10.7%
Found: C, 61.7; H, 5.2; N, 10.6%.

Example 2

*N*-[[[1-[(6-Fluoro-2-naphthyl)methyl]-4-piperidinyl]amino]carbonyl]-4-pyridinecarboxamide

A mixture of 4-amino-1-[(6-fluoro-2-naphthyl)methyl]piperidine (1.3 g, 5 mmol), 4-pyridoylurea (0.9 g, 5 mmol) and pyridine (6 ml) was stirred at reflux for 16 hours. The solution was diluted with water (8 ml) and the precipitated dark brown product collected by filtration to give 1.5 g. The crude product was dissolved in chloroform (25 ml), shaken with decolourising charcoal, filtered and evaporated. The residue was suspended in hot ethanol (20 ml) and succinic acid (0.44 g) added to give a clear solution which crystallised on standing. The product crystals were collected by filtration and washed with ethanol to give the succinate salt of the title compound. m.p. 187—188°C.

Analysis

$C_{23}H_{23}N_4FO_2 \cdot C_4H_6O_4$ requires: C, 61.82; H, 5.57; N, 10.68%
Found: C, 61.89; H, 5.66; N, 10.51%.

Example 3

The compound of example 1 when in the form of the free base has an m.p. 141—142°C. The monohydrochloride $\frac{3}{4}$ $H_2O$ salt has an m.p. 242—44°C.

Example 4

N-[[[-1-[(6-Fluoro-2-naphthyl)methyl]-4-piperidinyl]amino]carbonyl]-3-pyridinecarboxamide

A solution of 3-nicotinoylisocyanate (1.55 g, 5% excess) in $CH_2Cl_2$ (10 ml) was added dropwise to a stirred solution of 4-amino-1-[(6-fluoro-2-naphthyl)methyl]piperidine (2.6 g, 10 mmol) in $CHCl_2$ (50 ml) protected from atmospheric moisture. After addition was completed the reaction was stirred for a further 1 hour then evaporated. The residue was dissolved in dimethylformamide (5 ml) and methanesulphonic acid (0.8 g) in isopropanol (5 ml) added to precipitate the methanesulphonate salt, m.p. 227—228°C.

Analysis

$C_{23}H_{23}N_4FO_2 \cdot CH_4O_3S \cdot 0.25\ H_2O$ requires: C 56.85; H, 5.47; N, 11.05%
Found: C, 56.68; H, 5.40; N, 11.37%.

Example 5

N-[[[-1-[(6-Fluoro-2-naphthyl)methyl]-4-piperidinyl]amino]carbonyl]-3-pyridinecarboxamide

A mixture of nicotinoyl chloride (3.1 g, 22 mmol) N-[[[(6-fluoro-2-naphthyl)methyl]-4-piperidinyl]urea (6.02 g, or 20 mmol), dry pyridine (2.5 ml) and 1,2-dichloroethane (30 ml) is stirred and heated at reflux for 16 hours. The solution is then cooled, washed with aqueous sodium carbonate solution, dried and evaporated. The residue is crystallised from ethanol and treated with maleic acid to give the title maleate, m.p. 206—207°C.

Example 6

N-[[[-1-[(6-Fluoro-2-naphthyl)methyl]-4-piperidinyl]amino]carbonyl]-3-pyridinecarboxamide

2-Bromomethyl-6-fluoronaphthalene (12 g, 50 mmol) was added in one portion to a solution of N-[[[4-pyridyl]-amino]carbonyl]-3-pyridinecarboxamide (12.1 g, 50 mmol) in dimethylformamide (50 ml). The mixture was stirred for 2 hours and then diluted with water (100 ml) to precipitate *N*-[[[1-[(6-fluoro-2-naphthylmethyl]-4-pyridinium]amino]carbonyl]-3-pyridinecarboxamide bromide (12 g, 50%).

The above product was suspended in isopropanol (100 ml), sodium borohydride (6 g, 180 mmol) added and the mixture stirred at reflux for 16 hours. The solvent was evaporated and the residue diluted with water. The precipitate was collected by filtration and crystallised from ethanol.

**Claims for the Contracting States: BE CH DE FR IT LI LU NL SE**

1. A compound of formula

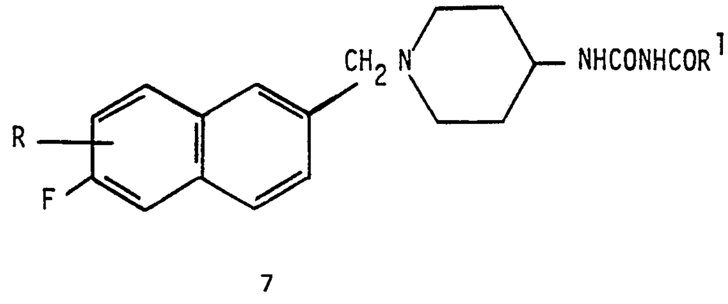

or a salt thereof, wherein R is hydrogen or fluorine, and $R^1$ represents 3-pyridyl or 4-pyridyl.

2. N-[[[1-[(6-Fluoro-2-naphthyl)methyl]-4-piperidinyl]amino]carbonyl]-3-pyridinecarboxamide or a pharmaceutically acceptable salt thereof.

3. N-[[[1-[(6-Fluoro-2-naphthyl)methyl]-4-piperidinyl]amino]carbonyl]-4-pyridinecarboxamide or a pharmaceutically acceptable salt thereof.

4. A compound as claimed in any one of Claims 1 and 3 in the form of the maleate salt.

5. A process for preparing a compound of formula I as claimed in Claim 1 or a salt thereof, which comprises

(a) reacting a compound of formula

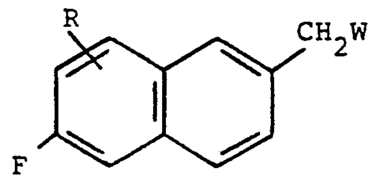

(III)

where R is as defined above and W represents a leaving group or a radical of formula $OSO_2OR^2$ where $R^2$ is

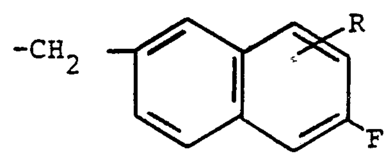

with a compound of formula IIIa

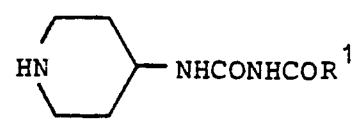

(IIIa)

where $R^1$ is as defined above;

(b) reacting a compound of formula

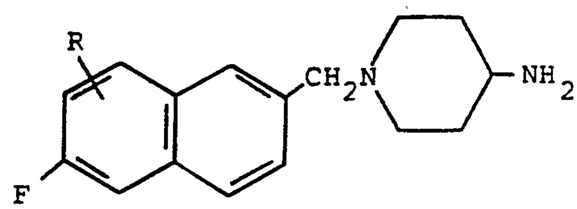

(IV)

wherein R is as defined above, with a compound of formula

$OCNCOR^1$ (IVa)

or a compound of formula

$H_2NCONHCOR^1$ (IVb)

wherein $R^1$ is as defined above, or

(c) acylating a compound of formula

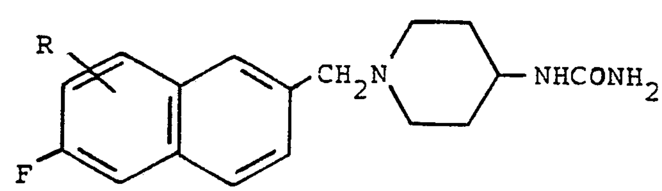

(V)

wherein R is as defined above, with an acylating agent containing the group

$$—COR^1$$

or

(d) reducing a compound of formula

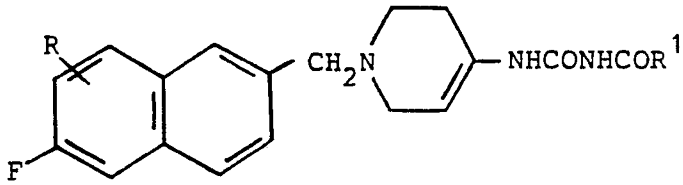

(VI)

or

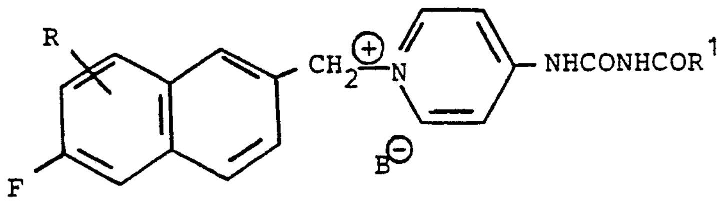

(VII)

or

(e) reacting a compound of formula

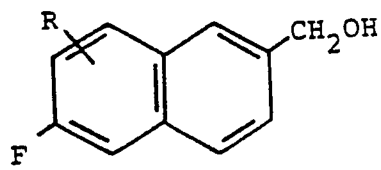

(VIII)

wherein R is as defined above with a compound of formula IIIa as defined above in the presence of a nickel catalyst; or

(f) converting a basic compound of formula I to an acid addition or quaternary ammonium salt; or

(g) converting an acid addition salt of a compound of formula I to the free base form.

6. A process (a) as claimed in Claim 5 wherein W is bromine or chlorine.

7. A process (c) as claimed in Claim 5 wherein the acylating agent is an acid halide, anhydride, mixed anhydride or active ester.

8. A process (d) as claimed in Claim 5 wherein the reduction is carried out using an alkali metal borohydride in a secondary alkanol of 3 to 5 carbon atoms.

9. A process (g) as claimed in Claim 5 in which the free base form is acidified with maleic acid to give the maleate salt.

10. A pharmaceutical composition comprising a compound of formula I as defined in Claim 1 or a pharmaceutically acceptable acid addition or quaternary ammonium salt thereof and a pharmaceutically acceptable carrier.

11. A composition as claimed in Claim 10 which is in the form of tablets or capsules.

12. A composition as claimed in Claim 10 or Claim 11 in which the compound of formula I is N-[[[1-[(6-fluoro-2-naphthyl)methyl]-4-piperidinyl]aminocarbonyl]-3-pyridinecarboxamide, or N-[[[1-[6-fluoro-2-naphthyl)methyl]-4-piperidinyl]amino]carbonyl]-4-pyridinecarboxamide or a pharmaceutically acceptable salt thereof.

**Claims for the Contracting States: AT GR ES**

1. A process for preparing a compound of formula

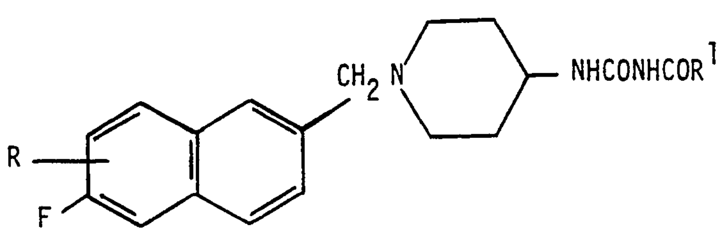

(I)

or a salt thereof, wherein R represents hydrogen or fluorine and $R^1$ represents 3-pyridyl or 4-pyridyl which comprises

(a) reacting a compound of formula

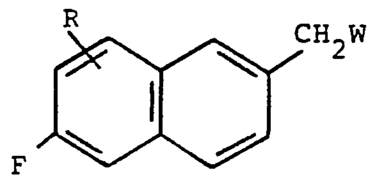

(III)

where R is as defined above and W represents a leaving group or a radical of formula $OSO_2OR^2$ where $R^2$ is

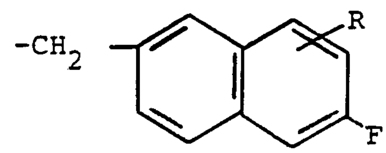

with a compound of formula IIIa

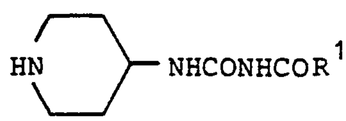

(IIIa)

where $R^1$ is as defined above;

(b) reacting a compound of formula

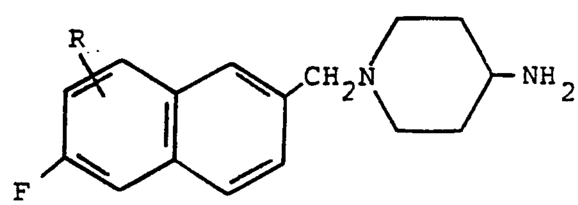

(IV)

wherein R is as defined above, with a compound of formula

$$OCNCOR^1 \qquad \text{(IVa)}$$

or a compound of formula

$$H_2NCONHCOR^1 \qquad \text{(IVb)}$$

wherein $R^1$ is as defined above, or

(c) acylating a compound of formula

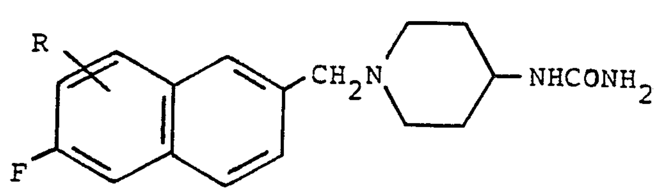

(V)

wherein R is as defined above, with an acylating agent containing the group

—COR[1]

or

(d) reducing a compound of formula

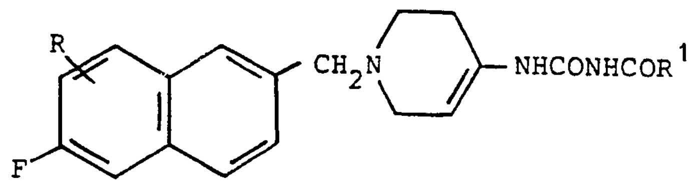

(VI)

or

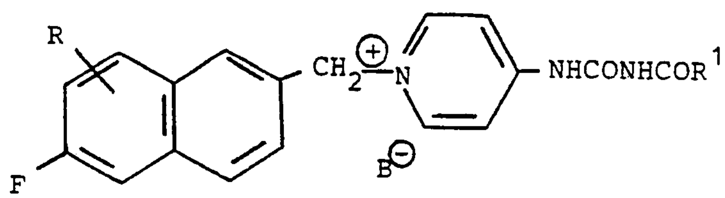

(VII)

or

(e) reacting a compound of formula

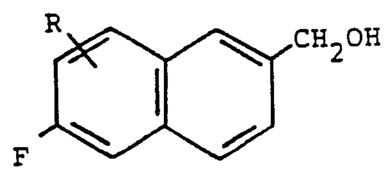

(VIII)

wherein R is as defined above with a compound of formula IIIa as defined above in the presence of a nickel catalyst; or

(f) converting a basic compound of formula I to an acid addition or quaternary ammonium salt; or

(g) converting an acid addition salt of a compound of formula I to the free base form.

2. A process (a) as claimed in Claim 1 wherein W is bromine or chlorine.

3. A process (c) as claimed in Claim 1 wherein the acylating agent is an acid halide, anhydride, mixed anhydride or active ester.

4. A process (d) as claimed in Claim 1 wherein the reduction is carried out using an alkali metal borohydride in a secondary alkanol of 3 to 5 carbon atoms.

5. A process (g) as claimed in Claim 1 in which the free base form is acidified with maleic acid to give the maleate salt.

6. A process as claimed in any one of Claims 1 to 4 in which the compound prepared is N-[[[1-[(6-fluoro-2-naphthyl)methyl]-4-piperidinyl]amino]carbonyl]-3-pyridinecarboxamide or a pharmaceutically acceptable salt thereof.

7. A process as claimed in any one of Claims 1 to 4 in which the compound prepared is N-[[[1-[(6-fluoro-2-naphthyl)methyl]-4-piperidinyl]amino]carbonyl]-4-pyridinecarboxamide or a pharmaceutically acceptable salt thereof.

8. A process as claimed in any one of Claims 1 to 5 in which the compound prepared is the maleate salt of N-[[[1-[(6-fluoro-2-naphthyl)methyl]-4-piperidinyl]amino]carbonyl-3-pyridinecarboxamide or a pharmaceutically acceptable salt thereof.

9. A process for producing a therapeutic composition exhibiting psychotropic activity characterised in that a compound of formula I as defined in Claim 1 or a pharmaceutically acceptable acid addition or quaternary ammonium salt thereof is brought into a form suitable for therapeutic administration.

10. A process as claimed in Claim 9 in which the therapeutic composition is produced in the form of tablets or capsules.

11. A process as claimed in Claim 9 or Claim 10 in which the compound of formula I is N-[[[1-[6-fluoro-2-naphthyl)methyl]-4-piperidinyl]aminocarbonyl]-3-pyridinecarboxamide, or N-[[[1-[6-fluoro-2-naphthyl)-methyl]-4-piperidinyl]amino]carbonyl]-4-pyridinecarboxamide or a pharmaceutically acceptable salt thereof.

**Patentansprüche für die Vertragsstaaten: BE CH DE FR IT LI LU NL SE**

1. Verbindung der Formel

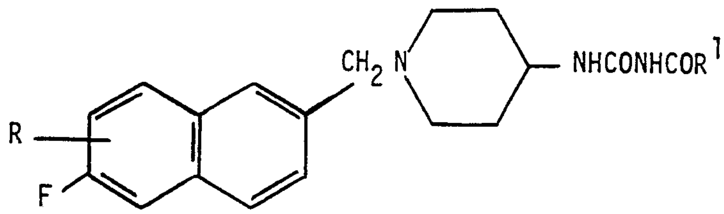

oder ein Salz hievon, worin R Wasserstoff oder Fluor ist und $R^1$ 3-Pyridyl oder 4-Pyridyl darstellt.

2. N-[[[1-[(6-Fluor-2-naphthyl)-methyl]-4-piperidinyl]amino]carbonyl]-3-pyridincarboxamid oder ein pharmazeutisch annehmbares Salz hievon.

3. N-[[[1-[(6-Fluor-2-naphthyl)-methyl]-4-piperidinyl]amino]carbonyl]-4-pyridincarboxamid oder ein pharmazeutisch annehmbares Salz hievon.

4. Verbindung nach einem der Ansprüche 1 und 3 in Form des Maleatsalzes.

5. Verfahren zur Herstellung einer Verbindung der Formel (I) nach Anspruch 1 oder eines Salzes hievon, das

(a) das Umsetzen einer Verbindung der Formel

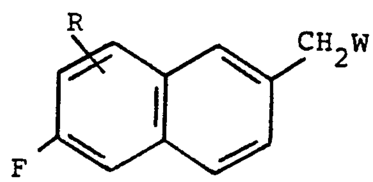

(III)

worin R wie oben definiert ist und W eine abspaltbare Gruppe oder einen Rest der Formel $OSO_2OR^2$ darstellt, wobei $R^2$

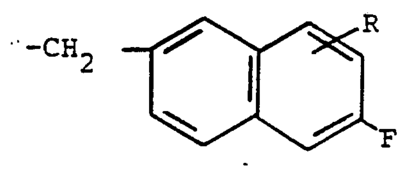

ist, mit einer Verbindung der Formel (IIIa)

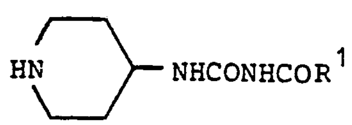

(IIIa)

worin $R^1$ wie oben definiert ist;

(b) das Umsetzen einer Verbindung der Formel

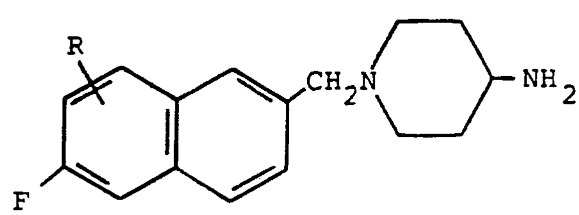

(IV)

worin R wie oben definiert ist, mit einer Verbindung der Formel

$OCNCOR^1$ (IVa)

oder einer Verbindung der Formel

$H_2NCONHCOR^1$ (IVb),

worin

$R^1$ wie oben definiert ist, oder

(c) das Acylieren einer Verbindung der Formel

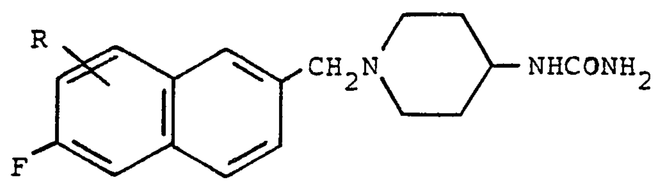

(V)

worin R wie oben definiert ist, mit einem Acylierungsmittel, das die Gruppe

$—COR^1$

enthält; oder

d) das Reduzieren einer Verbindung der Formel

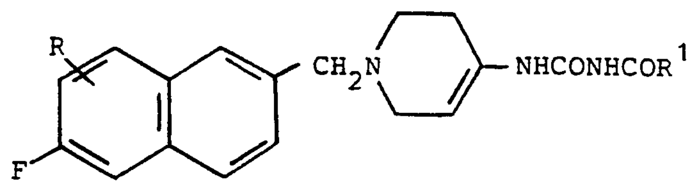

(VI)

oder

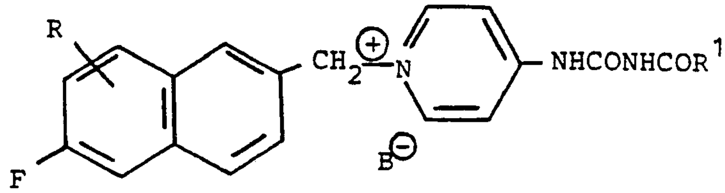

(VII)

oder

e) das Umsetzen einer Verbindung der Formel

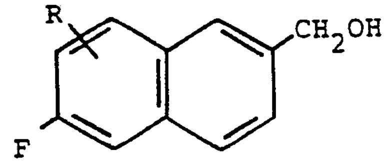

(VIII)

worin R wie oben definiert ist, mit einer Verbindung der Formel (IIIa), wie oben definiert, in Gegenwart eines Nickelkatalysators; oder

f) das Überführen einer basischen Verbindung der Formel (I) in ein Säureadditionssalz oder ein quartäres Ammoniumsalz; oder

g) das Überführen eines Säureadditionssalzes einer Verbindung der Formel (I) in die freie Basenform umfaßt.

6. Verfahren (a) nach Anspruch 5, worin W Brom oder Chlor ist.

7. Verfahren (c) nach Anspruch 5, worin das Acylierungsmittel ein Säurehalogenid, Anhydrid, gemischtes Anhydrid oder ein aktiver Ester ist.

8. Verfahren (d) nach Anspruch 5, worin die Reduktion unter Verwendung eines alkalimetallborhydrids in einem sekundären Alkanol mit 3 bis 5 Kohlenstoffatomen durchgeführt wird.

9. Verfahren (g) nach Anspruch 5, worin die freie Basenform mit Maleinsäure angesäuert wird, wobei das Maleatsalz erhalten wird.

10. Pharmazeutische Zusammensetzung, die eine Verbindung der Formel (I), wie in Anspruch 1 definiert, oder ein pharmazeutisch annehmbares Säureadditionssalz oder quartäres Ammoniumsalz hievon und einen pharmazeutisch annehmbaren Träger umfaßt.

11. Zusammensetzung nach Anspruch 10 in Form von Tabletten oder Kapseln.

12. Zusammensetzung nach Anspruch 10 oder 11, worin die Verbindung der Formel I N - [[[1 - [(6 - Fluor - 2 - naphthyl) - methyl] - 4 - piperidinyl] - aminocarbonyl] - 3 - pyridincarboxamid oder N - [[[1 - (6 - Fluor - 2 - naphthyl) - methyl] - 4 - piperidinyl]amino] - carbonyl - 4 - pyridincarboxamid oder ein pharmazeutisch annehmbares Salz hievon ist.

**Patentansprüche für die Vertragsstaaten: AT ES GR**

1. Verfahren zur Herstellung einer Verbindung der Formel

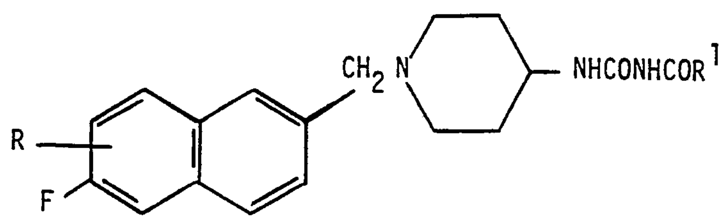

(I)

oder eines Salzes hievon, worin R Wasserstoff oder Fluor bedeutet und $R^1$ 3-Pyridyl oder 4-Pyridyl, das

(a) das Umsetzen einer Verbindung der Formel

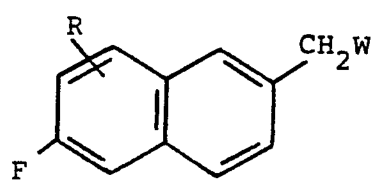

(III)

worin R wie oben definiert ist und W eine abspaltbare Gruppe oder einen Rest der Formel $OSO_2OR^2$ darstellt, wobei $R^2$

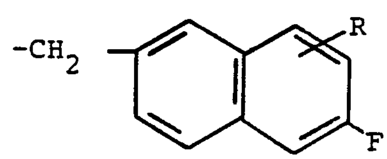

ist, mit einer Verbindung der Formel (IIIa)

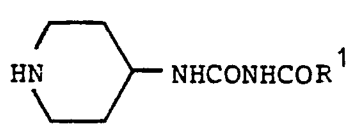

(IIIa)

worin $R^1$ wie oben definiert ist;

(b) das Umsetzen einer Verbindung der Formel

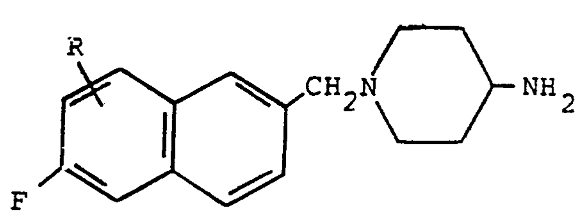

(IV)

worin R wie oben definiert ist, mit einer Verbindung der Formel

$OCNCOR^1$ (IVa)

oder einer Verbindung der Formel

$H_2NCONHCOR^1$ (IVb),

worin

$R^1$ wie oben definiert ist, oder

(c) das Acylieren einer Verbindung der Formel

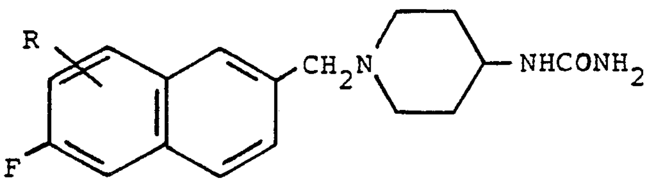

(V)

worin R wie oben definiert ist, mit einem Acylierungsmittel, das die Gruppe

$-COR^1$

enthält; oder

d) das Reduzieren einer Verbindung der Formel

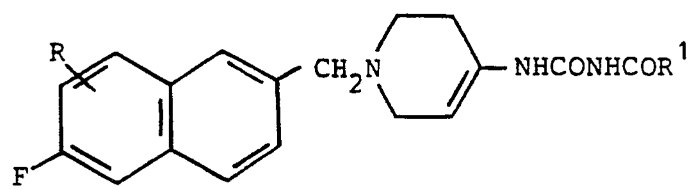

(VI)

oder

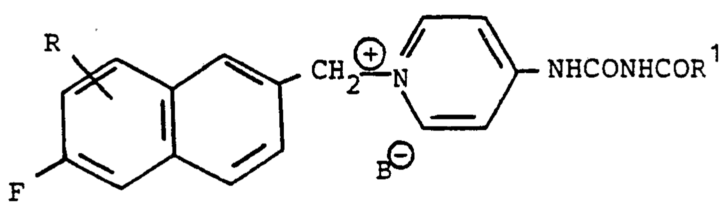

(VII)

oder

e) das Umsetzen einer Verbindung der Formel

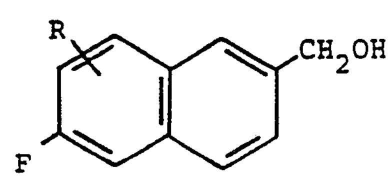

(VIII)

worin R wie oben definiert ist, mit einer Verbindung der Formel (IIIa), wie oben definiert, in Gegenwart eines Nickelkatalysators; oder

f) das Überführen einer basischen Verbindung der Formel (I) in ein Säureadditionssalz oder ein quartäres Ammoniumsalz; oder

g) das Überführen eines Säureadditionssalzes einer Verbindung der Formel (I) in die freie Basenform umfaßt.

2. Verfahren (a) nach Anspruch 1, worin W Brom oder Chlor ist.

3. Verfahren (c) nach Anspruch 1, worin das Acylierungsmittel ein Säurehalogenid, Anhydrid, gemischtes Anhydrid oder ein aktiver Ester ist.

4. Verfahren (d) nach Anspruch 1, worin die Reduktion unter Verwendung eines alkalimetallborhydrids in einem sekundären Alkanol mit 3 bis 5 Kohlenstoffatomen durchgeführt wird.

5. Verfahren (g) nach Anspruch 1, worin die freie Basenform mit Maleinsäure angesäuert wird, wobei das Maleatsalz erhalten wird.

6. Verfahren nach einem der Ansprüche 1 bis 4, worin die hergestellte Verbindung N-[[[1-[(6-Fluoro-2-naphthyl)-methyl]-4-piperidinyl]-amino]-carbonyl]-3-pyridincarboxamid oder ein pharmazeutisch annehmbares Salz hievon.

7. Verfahren nach einem der Ansprüche 1 bis 4, worin die hergestellte Verbindung N-[[[1-[(6-Fluoro-2-naphthyl)-methyl]-4-piperidinyl]-amino]-carbonyl]-4-pyridincarboxamid oder ein pharmazeutisch annehmbares Salz hievon.

8. Verfahren nach einem der Ansprüche 1 bis 5, worin die hergestellte Verbindung das Maleatsalz von N-[[[1-[(6-Fluor-2-naphthyl)-methyl]-4-piperidinyl]-amino]-carbonyl]-3-pyridincarboxamid oder ein pharmazeutisch annehmbares Salz hievon ist.

9. Verfahren zur Herstellung einer therapeutischen Zusammensetzung, die psychotrope Aktivität zeigt, dadurch gekennzeichnet, daß eine Verbindung der Formel (I), wie in Anspruch 1 definiert, oder ein pharmazeutisch annehmbares Säureadditionssalz oder quartäres Ammoniumsalz hievon in eine für therapeutische Verabreichung geeignete Form gebracht wird.

10. Verfahren nach Anspruch 9, worin die therapeutische Zusammensetzung in Form von Tabletten von Kapseln hergestellt wird.

11. Verfahren nach Anspruch 9 oder 10, worin die Verbindung der Formel (I) N - [[[1 - [(6 - Fluor - 2 - naphthyl) - methyl] - 4 - piperidinyl] - amino - carbonyl] - 3 - pyridincarboxamid oder N - [[[1 - [(6 - Fluor - 2 - naphthyl) - methyl] - 4 - piperidinyl] - amino] - carbonyl] - 4 - pyridincarboxamid oder ein pharmazeutisch annehmbares Salz hievon ist.

**Revendications pour les Etats contractants: BE CH DE FR IT LI LU NL SE**

1. Composé de formule

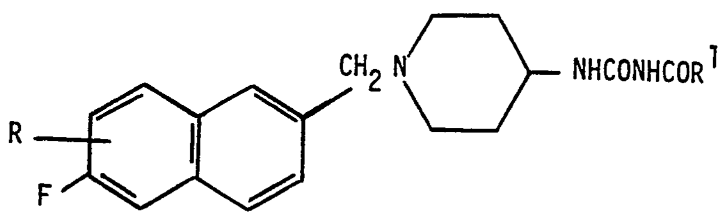

ou un sel de ce composé, formule dans laquelle R est l'hydrogène ou le fluor, et $R^1$ représente le groupe 3-pyridyle ou 4-pyridyle.

2. Le N-[[[1-[(6-fluoro-2-naphtyl)méthyl]-4-pipéridinyl]amino]carbonyl]-3-pyridinecarboxamide ou un sel pharmaceutiquement acceptable de ce composé.

3. Le N-[[[1-[(6-fluoro-2-naphtyl)méthyl]-4-pipéridinyl]amino]carbonyl]-4-pyridinecarboxamide ou un sel pharmaceutiquement acceptable de ce composé.

4. Composé suivant l'une quelconque des revendications 1 et 3, sous la forme du sel d'acide maléique.

5. Procédé de préparation d'un composé de formule I suivant la revendication 1 ou d'un sel de ce composé, qui consiste

(a) à faire réagir un composé de formule

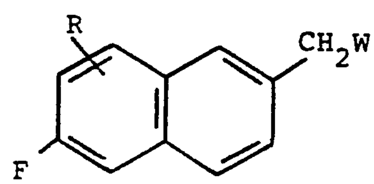

(III)

dans laquelle R est tel que défini ci-dessus et W représente un groupe partant un ou radical de formule $OSO_2OR^2$, dans laquelle $R^2$ est un groupe

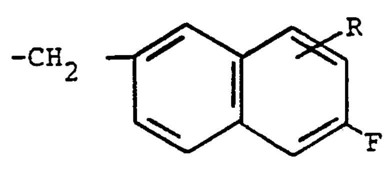

avec un composé de formule IIIa

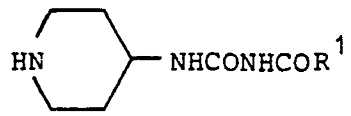

(IIIa)

dans laquelle $R^1$ est tel que défini ci-dessus;

(b) à faire réagir un composé de formule

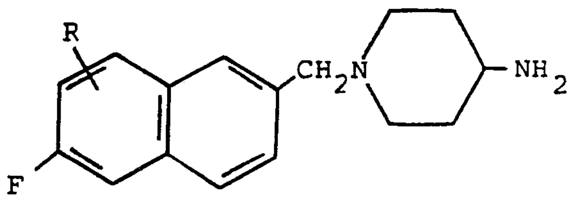

(IV)

dans laquelle R est tel que défini ci-dessus, avec un composé de formule

$OCNCOR^1$ (IVa)

ou un composé de formule

$H_2NCONHCOR^1$ (IVb)

dans laquelle $R^1$ est tel ue défini ci-desus, ou

(c) à acyler un composé de formule

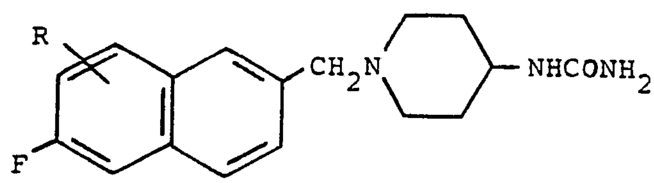

(V)

dans laquelle R est que défini ci-dessus, avec un agent acylant contenant le groupe

$—COR^1$

ou

(d) à réduire un composé de formule

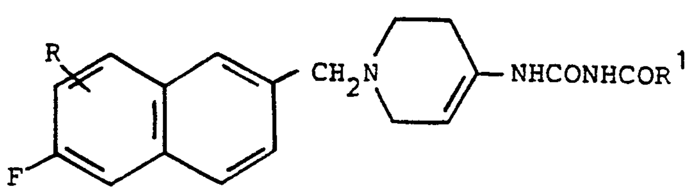

(VI)

or

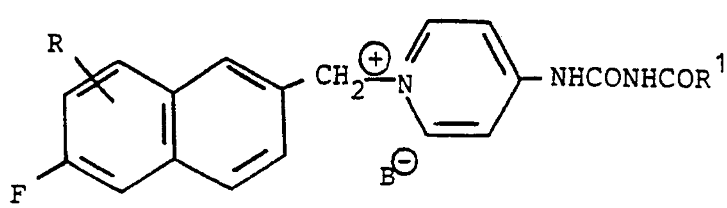

(VII)

oun

(e) à faire réagir un composé de formule

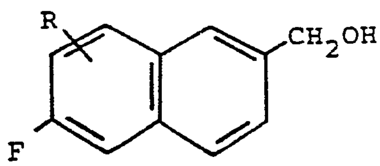

(VIII)

dans laquelle R est tel que défini ci-dessus, avec un composé de formule IIIa tel que défini ci-dessus en présence d'un catalyseur au nickel; ou

(f) à convertir un composé basique de formule I en un sel d'addition d'acide ou d'ammonium quaternaire; ou bien

(g) à convertir un sel d'addition d'acide d'un composé de formule I en la forme base libre.

6. Procédé (a) suivant la revendication 5, dans lequel W est le brome ou le chlore.

7. Procédé (c) suivant la revendication 5, dans lequel l'agent acylant est un halogénure d'acide, un anhydride d'acide, un anhydride mixte ou un ester actif.

8. Procédé (d) suivant la revendication 5, dans lequel la réduction est effectuée en utilisant un borohydrure de métal alcalin dans un alcanol secondaire ayant 3 à 5 atomes de carbone.

9. Procédé (g) suivant la revendication 5, dans lequel la forme base libre est acidifiée avec l'acide maléique en donnant le maléate.

10. Composition pharmaceutique, comprenant un composé de formule I tel que défini dans la revendication 1 ou un sel d'addition d'acide ou un sel d'ammonium quaternaire pharmaceutiquement acceptable de ce composé, et un support acceptable du point de vue pharmaceutique.

11. Composition suivant la revendication 10, qui est sous la forme de comprimés ou de gélules.

12. Composition suivant la revendication 10 ou la revendication 11, dans laquelle le composé de formule I est le N - [[[1 - [(6 - fluoro - 2 - naphtyl)méthyl] - 4 - pipéridinyl]amino]carbonyl] - 3 - pyridinecarboxamide ou le N - [[[1 - [6 - fluoro - 2 - naphtyl)méthyl] - 4 - pipéridinyl] - amino]-carbonyl] - 4 - pyridine]carboxamide ou un sel pharmaceutiquement acceptable de ce composé.

**Revendications pour les Etats contractants: AT ES GR**

1. Procédé de préparation d'un composé de formule

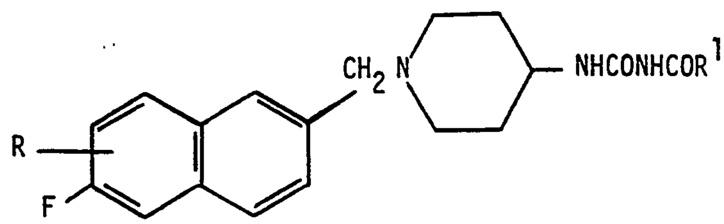

(I)

ou d'un sel de ce composé, formule dans laquelle R est l'hydrogène ou le fluor, et $R^1$ représente le groupe 3-pyridyle ou 4-pyridyle, qui consiste

(a) à faire réagir un composé de formule

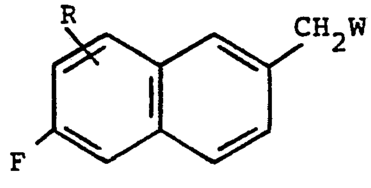

(III)

dans laquelle R est tel que défini ci-dessus et W représente un groupe partant un ou radical de formule $OSO_2OR^2$, dans laquelle $R^2$ est un groupe

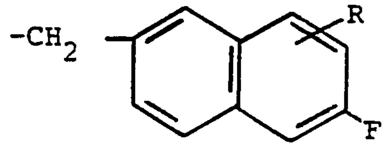

avec un composé de formule IIIa

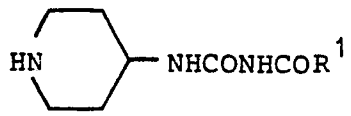

(IIIa)

dans laquelle $R^1$ est tel que défini ci-dessus;

(b) à faire réagir un composé de formule

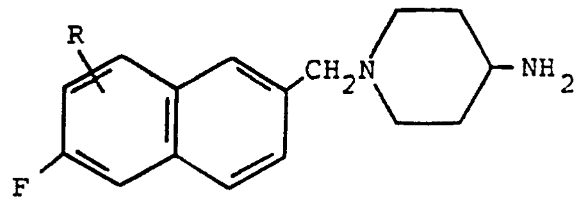

(IV)

dans laquelle R est tel que défini ci-dessus, avec un composé de formule

$OCNCOR^1$ (IVa)

ou un composé de formule

$H_2NCONHCOR^1$ (IVb)

où $R^1$ est tel ue défini ci-dessus, ou

(c) à acyler un composé de formule

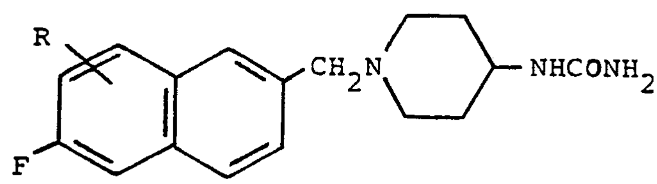

(V)

dans laquelle R est que défini ci-dessus, avec un agent acylant contenant le groupe

$—COR^1$

ou bien

(d) à réduire un composé de formule

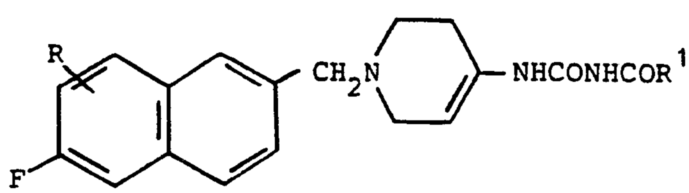

(VI)

or

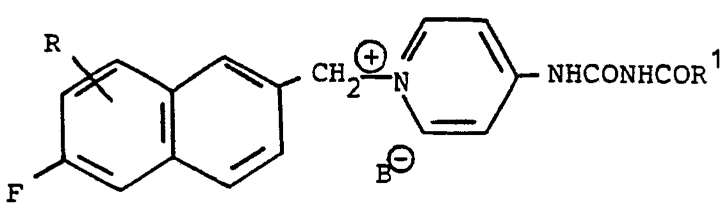

(VII)

ou

(e) à faire réagir un composé de formule

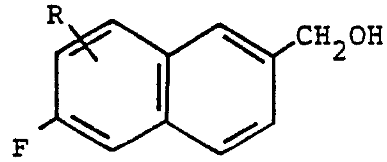

(VIII)

dans laquelle R est tel que défini ci-dessus, avec un composé de formule IIIa tel que défini ci-dessus en présence d'un catalyseur au nickel; ou bien

(f) à convertir un composé basique de formule I en un sel d'addition d'acide ou d'ammonium quaternaire; ou bien

(g) à convertir un sel d'addition d'acide d'un composé de formule I en la forme base libre.

2. Procédé (a) suivant la revendication 1, dans lequel W est le brome ou le chlore.

3. Procédé (c) suivant la revendication 1, dans lequel l'agent acylant est un halogénure d'acide, un anhydride d'acide, un anhydride mixte ou un ester actif.

4. Procédé (d) suivant la revendication 1, dans lequel la réduction est effectuée en utilisant un borohydrure de métal alcalin dans un alcanol secondaire 3 à 5 atomes de carbone.

5. Procédé (g) suivant la revendication 1, dans lequel la forme base libre est acidifiée avec l'acide maléique en donnant le maléate.

6. Procédé suivant l'une quelconque des revendications 1 à 4, dans lequel le composé préparé est le N-[[[1-[(6-fluoro-2-naphtyl)méthyl]-4-pipéridinyl]amino]carbonyl]-3-pyridinecarboxamide ou un sel pharmaceutiquement acceptable de ce composé.

7. Procédé suivant l'une quelconque des revendications 1 à 4, dans lequel le composé préparé est le en N-[[[1-[(6-fluoro-2-naphtyl)méthyl]-4-pipéridinyl]amino]carbonyl]-4-pyridinecarboxamide ou un sel pharmaceutiquement acceptable de ce composé.

8. Procédé suivant l'une quelconque des revendications 1 à 5, dans lequel le composé préparé est le sel d'acide maléique du N - [[[1 - [(6 - fluoro - 2 - naphtyl)méthyl] - 4 - pipéridinyl]amino]carbonyl] - 3 - pyridinecarboxamide ou un sel pharmaceutiquement acceptable de ce composé.

9. Procédé de préparation d'une composition thérapeutique douée d'activité psychotrope, caractérisé en ce qu'un composé de formule I, tel que défini dans la revendication 1 ou un sel d'addition d'acide ou un sel d'ammonium quaternaire pharmaceutiquement acceptable de ce composé est mis sous une forme qui convient pour l'administration thérapeutique.

10. Procédé suivant la revendication 9, dans lequel la composition thérapeutique est produite sous la forme de comprimés ou de gélules.

11. Procédé suivant la revendication 9 ou la revendication 10, dans lequel le composé de formule I est le N - [[[1 - [(6 - fluoro - 2 - naphtyl)méthyl] - 4 - pipéridinyl]amino]carbonyl] - 3 - pyridinecarboxamide ou le N - [[[1 - [(6 - fluoro - 2 - naphtyl)méthyl] - 4 - pipéridinyl]amino]carbonyl] - 4 - pyridinecarboxamide ou un sel pharmaceutiquement acceptable de ce composé.